(19) Europäisches Patentamt / European Patent Office / Office européen des brevets

(11) **EP 2 731 705 B1**

## (12) EUROPEAN PATENT SPECIFICATION

(45) Date of publication and mention
of the grant of the patent:
**28.10.2015 Bulletin 2015/44**

(21) Application number: **12733036.3**

(22) Date of filing: **27.06.2012**

(51) Int Cl.:
*B01J 19/00* (2006.01)　　　*G01N 33/18* (2006.01)
*C12M 1/00* (2006.01)　　　*B01J 4/00* (2006.01)
*B01J 4/02* (2006.01)

(86) International application number:
**PCT/EP2012/062460**

(87) International publication number:
**WO 2013/010764 (24.01.2013 Gazette 2013/04)**

(54) **METHODS AND APPARATUS FOR ANALYSIS OF AQUATIC CHEMICAL AND/OR BIOLOGICAL SYSTEMS**

VERFAHREN UND VORRICHTUNG ZUR ANALYSE VON AQUATISCHEN CHEMISCHEN UND/ODER BIOLOGISCHEN SYSTEMEN

PROCÉDÉ ET APPAREIL POUR L'ANALYSE DE SYSTÈMES CHIMIQUES ET/OU BIOLOGIQUES AQUATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2011 GB 201112269**

(43) Date of publication of application:
**21.05.2014 Bulletin 2014/21**

(73) Proprietor: **Université de Mons**
**7000 Mons (BE)**

(72) Inventors:
• **GROSJEAN, Philippe**
**B-1410 Waterloo (BE)**

• **LEBLUD, Julien**
**B-7334 Hautrage (BE)**
• **BATIGNY, Antoine**
**F-59300 Valenciennes (FR)**

(74) Representative: **Farmer, Guy Dominic**
**ARC-IP sprl**
**Rue Emile Francqui, 4**
**1435 Mont-Saint-Guibert (BE)**

(56) References cited:
**WO-A1-2010/110773　　CN-A- 101 709 263**
**US-A1- 2004 024 493**

**Description**

The present invention relates to methods and apparatus for analysis of liquid, particularly aquatic, chemical and/or biological systems.

Most "traditional" chemostats are bioreactors that operate like a continuous stirred-tank reactor (CSTR). A given volume of liquid is contained in a well-stirred reactor unit. Biomass is produced in this liquid medium (bacteria, algae, cells in culture, etc.). The system is rendered stable in time by: (1) eliminating excess of the produced biomass and by-products, and (2) by adding chemicals that are consumed by the living organisms (nutriments, oxygen, food, etc.). There is a continuous inlet of additives and a corresponding outlet of products (since the volume of the reactor is constant, inlet and outlet flow rates are equal). Both the flow rate of these inlet/outlet and the composition of the inlet water are adjusted in such a way that the chemical composition of the water inside the stirred reactor remains stable. This is done automatically by measuring the quality of the water in the outlet, and by adjusting the amount of additives accordingly. Usually, the biomass is also maintained constant. This is regulated by measuring the density of cells in the reactor and by changing the inlet/outlet flow rate to more or less dilute the culture medium until biomass stabilizes at the desired level inside the reactor (so-called regime state of the bioreactor).

Such a system is convenient and efficient to produce living biomass and allows for some calculation of the mass balance for given chemicals, which in turn, are proxies to estimate biological processes like growth, respiration, photosynthesis, etc. The mass balance for a given chemical, for example $O_2$, referenced as C in a continuous ideally stirred-tank reactor is:

$$\frac{dC}{dt} = F_{in} \cdot [C]_{in} - F_{out} \cdot [C]_{out} + \sum_i V_{reactor} \cdot v_i \cdot r_i$$

where $F_{in}$ and $F_{out}$ are inlet and outlet flow rates, respectively, $[C]_{in}$ and $[C]_{out}$ are concentrations of the considered chemical in the inlet and outlet[1], $V_{reactor}$ is the volume of the reactor unit, $v_i$ is the stoichiometric coefficient of C in the ith reaction that occur in the chemostat and $r_i$ is the reaction rate for that ith reaction. In the case of a constant volume reactor, $F_{in} = F_{out}$ and since we have a chemostat, the chemical is not supposed to vary significantly in time, that is:

$$\frac{dC}{dt} \simeq 0$$

(assuming the automatic regulation system is efficient, of course).
[1] Note that for gas, it is not the concentration, but the partial pressure that is considered in the reactions. We should really read here $P_{O2}$ instead of $[O_2]$ in this particular case, but that does not significantly change the explanation.

The equation simplifies to:

$$F_{in} \cdot \left( [C]_{out} - [C]_{in} \right) \simeq \sum_i V_{reactor} \cdot v_i \cdot r_i$$

In the last equation, the left member is a balance of input and output of C in the system, and the right member is a balance of all the reactions that occur inside the chemostat, that is, a quantification of the net flux of C between the living organisms that grow in the chemostat and the surrounding water. For instance, for organisms that only respire (no photosynthesis), the balance of oxygen input and output is a good estimation of the respiration rate of the living organisms inside the chemostat. It is thus possible to estimate respiration by using three measurements made on inlet and outlet waters: $F_{in}$, $[C]_{in}$ and $[C]_{out}$. The concentration of chemicals can be determined in various ways (electrochemistry, colorimetry, etc.). In the case of oxygen, this can be done using Clark oxygen electrodes, or optodes, placed on the inlet and outlet waters, and connected to oxymeters. Measuring the flow rate is a little bit more delicate. There are various systems and they are either rather expensive, or relatively inaccurate. Another option would be to use a very accurate constant flow pump (very expensive, and also not very convenient to regulate the flow, and thus the biomass inside the reactor). So, in practice, we observe one source of error that is common to all chemostats and all chemicals we would like to study: the error made in the estimation of the inlet/outlet flow rate.

Now, the equations we have developed above are only valid for continuous ideally stirred-tank reactors, that is, reactors where we have perfect and instantaneous mixing of the inlet water and the liquid inside the reactor. Real systems are not ideal because mixing occurs in a measurable amount of time. Since outlet is synchronous to inlet, it becomes hard to tell exactly how much of the additions through the inlet is eliminated through the outlet before even getting a chance to be in contact with the living organisms. Also, $[C]_{out}$ is not a good approximation any more of $[C]_{inside}$ (inside the reactor). Integration over time of the equations that describe a real system that is not ideally perfectly mixed is difficult and one must consider many additional parameters related to fluid dynamic and the way mixing is done inside the reactor. So, in practice, only the equations for an ideal reactor are usable but then, errors are introduced, and that lead to a bias in the quantification of the biological process in the reactor. The importance of the bias is dependent upon the residence time of the chemical inside the reactor versus the mixing time. If the former is 5-10 times larger than the latter, approximation is considered valid for engineering (i.e., industrial production of biomass). For research, where unbiased estimation of the processes is targeted, this can remain a problem.

WO 2010/110773 A1 relates to a "Methanogenic reactor", US 2004/0024493 A1 discloses a "Method, system and sub-system, for processing a chemical reaction" and CN 101 709 263 A relates to "Chemostat continuous cultivation device".

One aim of the present invention is thus to provide a practical and economically viable way to achieve analysis of liquid, particularly aquatic, chemical and/or biological systems with a desirably level of analytical accuracy.

According to one of its aspects, the present invention provides a method of analysing behaviour of a chemical and/or biological sample in a liquid medium, for example a liquid test medium, as defined by claim 1. According to another of its aspects, the present invention provides apparatus, for example a test apparatus, for analysing behaviour of a system, for example a test system, comprising a chemical and/or biological sample in a liquid medium as defined by claim 7. Alternative and/or preferred embodiments of the invention are defined in the dependent claims.

Based on the realisations that: (1) flow rate is a major source of error in chemostats and (2) biases occur in real chemostats due to lack of instant perfect mixing, we have designed a chemically stabilized reactor that operates differently. Instead of constant inlet and outlet, these are intermittent. The volume of the reactor is variable, so that it is possible to add supplementation (referred to below as a compensation liquid) without allowing any liquid to flow out of the reactor in a first stage. Then, the whole (the fluid in the reactor and the addition) is mixed during enough time to homogenize the content inside the reactor. Only then, a similar volume of the mix is eliminated from the reactor, so that the initial volume is recovered and by-products are eliminated. That way, our system behaves like an ideal, but intermittent stirred-tank reactor.

Both the amount of addition and the amount of liquid that is eliminated are weighted. Laboratory scales are widespread and allow for very accurate determination of the weight of liquid added, and then, eliminated from the reactor. Another benefit is that concentration expressed in term of weight, i.e., molality, or molinity, are temperature invariant. Concentration of the same chemical expressed in term of volume (which naturally derives from flow rate measurements in volume per time unit), i.e. molarity, varies with temperature. The source of error is low, but can be significant when very accurate determinations are required.

Variable volume may be achieved by using a gas compartment on top of the liquid in the reactor compartment whose volume can fluctuate. The role of the gas compartment is not limited to this purpose. It is also a compartment that may simulate atmosphere on top of the aquatic system in the chemostat, and thus, air-water gas exchanges may also be simulated in the unit. Furthermore, a gas compartment may be used to regulate oxygen partial pressure inside the reactor, and/or the partial pressure of one or more other gases. Since the unit operates intermittently, it is a closed unit during a significant period of time. During that stage, water chemistry is modified inside the reactor, as well as, exchanges between the liquid and gas compartments. To be considered as a chemostat, fluctuations must be reasonably small (say, no more than 5% of the concentration of the chemicals), and be readily equilibrated once liquid additions occur. This can be achieved by choosing carefully both the biomass per reactor volume and the time between two additions. Sometimes, buffer effects help to stabilize the medium. For example, pH is well buffered in seawater and changes less rapidly due to accumulation of $CO_2$ from respiration (in the example chemostat we discuss here) than the oxygen is consumed. In one litre of seawater, there is 5 to 7mg of oxygen dissolved; this can be very rapidly depleted when living organisms respire in a closed unit. In the system according to the invention, a gas compartment containing air may allow storage of much more oxygen in the whole system: at 1 atmosphere and 20°C, there is roughly 240mg of oxygen in one litre of air. Thus, a system made of 1L of seawater and 1L of air on top contains roughly 246mg of oxygen in total. The same reactor entirely filled with seawater contains only 12mg of oxygen, I.e. more than 20 times less. If air-water gas exchanges are fast enough (for instance, both the liquid and the gas are well stirred), oxygen concentration will be much more stable in the reactor with 1 L or seawater and 1L of air, for the same biomass of living organisms.

The preferred system according to the invention contains both a liquid and a gas phase that simulates air-water gas exchanges, and allows for a more stable oxygen concentration (or other gaseous components) in the water. Stabilisation of oxygen in the chemostat can also be mediated by intermittent replacement of the gas phase, simultaneous to the partial replacement of the liquid phase. The latter operates intermittently, with additions and eliminations of liquids being decoupled in time so as to make sure the medium is well mixed before eliminating any liquid. There is thus no bias in the equations used to quantify chemical fluxes. Finally, the unit does not require expensive fixed flow pumps, or accurate flow meters, because flow rates do not need to be measured. Liquids that are added or eliminated are discrete samples, and these samples can be accurately weighted, leading to much lower errors in the determination of inputs and outputs than in traditional chemostats. This design leads to a system that is optimized to quantify fluxes, including those that are usually hard to measure very accurately, like net flux of $CO_2$ in a buffered medium, e.g., seawater, in the presence of living organisms and thus, with many different processes that impact these fluxes (respiration, photosynthesis, calcification, nitrification, denitirification, etc.) combined with a rather complex chemical system (inorganic carbon species in seawater).

A non-limiting example of the invention is described below with reference to:

Fig 1 which is a schematic representation of an apparatus in accordance with the present invention;

Fig 2 which is a schematic representation showing a preferred operation cycle; and

Fig 3 which is a schematic representation of a possible 24 hour cycle arrangement.

The apparatus of the described embodiment may be thought of as a special kind of bioreactor that encloses a chemical or biological aquatic system in a unit which has its own enclosed atmosphere. Physico-chemical characteristics of the water compartment and of the atmosphere are stabilized and standardized. Any exchange between the water compartment and the chemical or biological system under study can be monitored, as well as all gas exchanges with the internal atmosphere in the unit. In the example, the system mainly targets changes in carbon chemical species, but can also be used to quantify exchanges of, say, nitrogen, phosphorus, silica, calcium, etc.

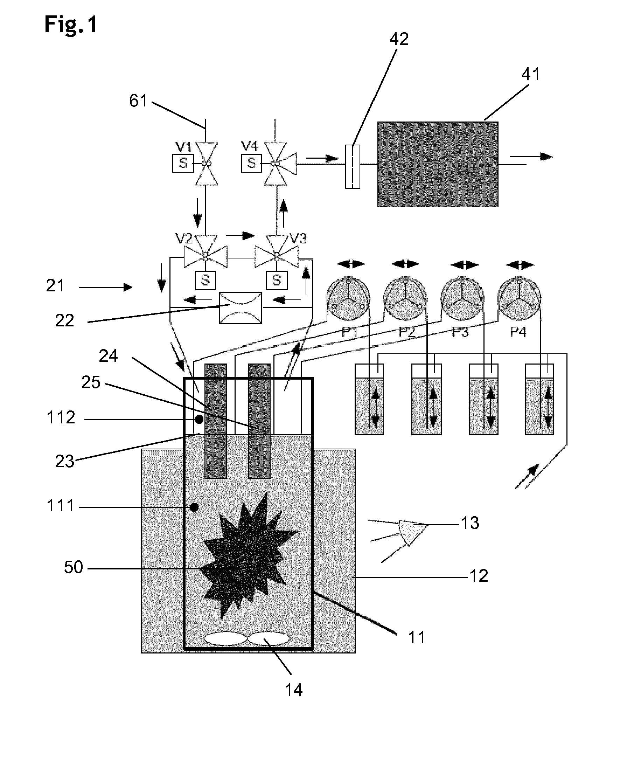

Fig 1 shows a general process and instrumentation diagram of an embodiment of a conglomerated test apparatus comprising a plurality of individual reaction vessels 11 (only one of which is shown) which each encloses a biological or chemical system under study 50 in water 111 and air 112 in a transparent container provided with a magnetic stirrer 14. A reaction vessel holder 12 in the form of a thermostated bath 12 retains each reaction vessel 11 under controllable artificial light 13 so that each reaction vessel is subjected to substantially identical, controlled external conditions. Each test apparatus comprises a gas circulation system 21 (in this example the gas is air of known composition) comprising a diaphragm pump 22 which moves the air inside the reaction vessel 11 and enhances gas exchanges at the water-air surface 23 and solenoid controlled pinch valves (V1, V2, V3 and V4). A peristaltic pump with autoclavable pipes can also replace the diaphragm pump 22 where sterile conditions are required.

Various optional instrumentations (here a pH probe 24 and an oxygen probe 25) continuously monitor the water which makes up the liquid test medium. Similarly, gas analyzers are connected to analyze the gas phase. In our example, an infrared gas analyzer (IRGA) 41 measures $CO_2$ partial pressure in an input gas 61 which may be selected from the reference air and the gas of the reaction vessel's internal atmosphere. A mechanical filter 42 protects the gas analyzer from contamination from water droplets, bacteria, etc.

Each test apparatus also comprises a series of addition/sampling peristaltic pumps (here P1-P4) which are configured and controlled to automatically add compensation solutions to and then collect samples from the liquid test medium 111 in the reaction vessel 11 (with intervening rinsing of the samples' containers).

The conglomerated unit comprises a plurality, typically greater than 2 and/or less than 12) test apparatus that run in parallel, each of which is selectively connectable to the gas analyzers, for example the IRGA 41, via an appropriate valve V4.

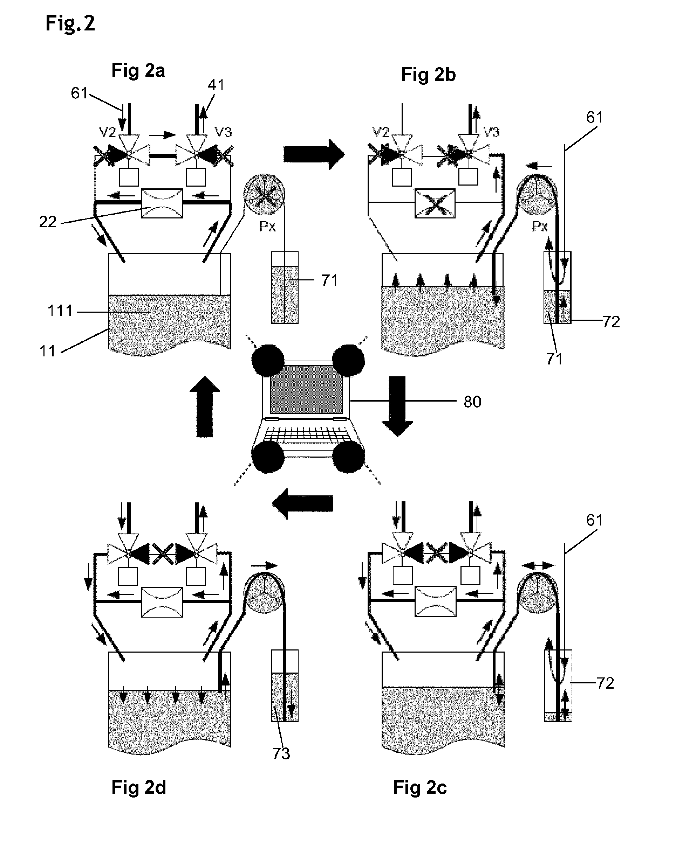

Fig 2 shows a schematic representation of a preferred operation cycle:

- During a reaction step, which runs for most of its operation cycle, the system is run as a closed unit (Fig 2a) in which valves V2 and V3 are configured so that previously introduced reference gas is circulated in a closed loop by diaphragm pump (DP) 22 so as to increase the contact with the liquid test medium 111 in the reaction vessel 11; reference gas from a reference gas source 61 is fed to the IRGA 41 (not shown) during this step so as to record a baseline with the gas analyzer.

- In an addition mode (Fig 2b) valve V3 prevents reference gas from reference gas source 61 being fed to the IRGA 41, diaphragm pump 22 is stopped so that there is no forced circulation of gas anymore inside the unit, but gas from inside the unit can freely flow to the IRGA 61, displaced by the addition of compensation solution 71 from the container 72 to the reaction vessel using one of the four peristaltic pump (Px) in the unit. Gas reference 61 can freely flow to container 72 during this step in order to avoid any vacuum during the operation. Compensation solution 71 is weighted before use.

- In a rinsing mode (Fig 2c) the peristaltic pump (Px) washes the combined compensation liquid vessel and liquid sample receiving vessel 72 with homogenized reaction liquid water 111 from the reaction vessel 11 thanks to the alternate operation of pump Px in one and the other direction. During this stage, reference gas 61 is fed in to the reaction vessel 11 at a flow rate high enough to obtain at the end the rinsing mode the same, or substantially the same chemical composition of the gas phase in the reaction vessel 11 as the reference gas. Reference gas 61 can also freely flow to container 72 in order to avoid any pressure difference on top of the liquid in it. Optionally, the diaphragm pump 22 can be run again to better mix the gas phase inside the reaction vessel 11.

- In a sampling mode (Fig 2d) a homogenized sample of the reaction liquid water 111 is collected, while the reference gas is still flushed inside the reaction vessel 11. Once this is completed, the unit is returned from this cycle which makes up a sampling step to its closed unit configuration for a subsequent reaction step. Liquid level in reactor vessel 11 is returned approximately the same value as in Fig 2a. The liquid sample 73 just collected can then be further weighted for accurate mass balance, and then analyzed with any suitable method in order to determine levels of chemical or biological items in the reaction vessel 11 at the end of the cycle (e.g., nutrients, alkalinity, dissolved organic carbon, nitrogen or phosphorus, bacteria, algae, etc.).

- Operation of the unit is controlled by a controller 80, preferably an appropriately configured computer.

In a prototype arrangement in which the biological sample being tested in the liquid test medium was a nubbin of the coral *Seriatopora hystrix*, the preferred duration of the steps is: closed unit reaction step (Fig 2a) 3 hours 50 minutes; addition step (Fig 2b) 4 minutes; rinsing step (Fig 2c) 2 minutes; sample extraction step (Fig 2d) 4 minutes.

The following is a more detailed description of the operation of the illustrated embodiment.

The test apparatus is a closed unit operating its reaction step most of the time, with valves V2 and V3 diverting reference gas to the gas analyzer, e.g., IRGA $CO_2$ analyzer 41. All peristaltic pumps (P1, P2, P3, P4) are off. The magnetic stirrer 14 homogenizes water inside the unit, and the diaphragm pump 22 mixes air inside the unit and enhances water-air gas exchanges. Water quality is monitored using the instrumentation 24, 25 installed inside reaction vessel 11.

After allowing a chemical and/or biological system 50 which is in contact with the liquid test medium to react for a predefined reaction step time (which is calculated to allow enough gas change (e.g., $O_2$ and $CO_2$ partial pressure) to quantify fluxes, but not too much so as to avoid significant physico-chemical changes (i.e., roughly 5-10% above or below the consign value would be acceptable values for most biological systems, but this is dependent on the particular experiment to run), a sampling step comprising a cycle of addition/sampling is initiated using one of the four P1-P4 peristaltic pumps. The sampling step runs as follows.

The unit is switched into its addition mode. The diaphragm pump 22 is switched off to limit water-air gas exchanges at this stage. Valve V3 is switched to open the gas phase of the unit to the IRGA $CO_2$ analyzer 41 and to block the source of the reference air 61. The selected peristaltic pump is activated in addition mode: the compensation solution 71 in the corresponding compensation liquid vessel is injected inside the reaction vessel 11. That solution 71 is formulated to precisely compensate chemical changes that occurred in the unit between two sampling steps. Since the volume of water in the reaction vessel 11 increases, it pushes air from the gas phase of the unit towards the IRGA $CO_2$ analyzer 41 (piston effect), which will measure $CO_2$ partial pressure of the gas phase in the unit after pipes and the infrared measurement cell are completely flushed (after 70-80 ml of compensation solution is injected in one prototype embodiment). The peristaltic pumps are configured to have a flow rate that allows injection of all the compensation solution over a duration which represents half to two-third of the time the pump is run in addition mode (two to three minutes in our prototype while the addition mode lasts for four minutes). In this way, we don't need accurate flow peristaltic pumps, and all of the compensation liquid is injected with a margin during which reference air from within the compensation liquid vessel is injected into the reaction vessel 11. Thus, it is not the flow rate of the pump that is used to determine how much of the compensation solution is injected, but the weighing of that solution before use (thus, a measurement that is at least one order of magnitude more accurate than an estimation from flow rate and time).

The unit is then switched into a rinsing mode for a duration of 2 minutes. The peristaltic pump is run three times during 20 seconds in one direction and during 20 seconds in the other direction. The bottom of the compensation liquid vessel is thus rinsed three times with the liquid test medium water from the reaction vessel 11. Meanwhile, the liquid test medium water and compensation solution are progressively homogenized in the reaction vessel unit thanks to the magnetic stirrer 14.

Re-equilibration of the internal atmosphere in the unit is also initiated at this stage: the diaphragm pump is switched on again and valve V2 is switched to feed reference air into the unit.

The unit is then configured in sample extraction mode, which lasts for another four minutes. The peristaltic pump is activated and combined compensation liquid vessel and sample receiving vessel 72 is completely filled with a sample 73 whose volume approximately equates the volume of the compensation solution that was added. This is assured by the collecting pipe inside the reaction vessel that plunges just deep enough in the unit to pump the correct volume of solution, but that is flushed with air once the water goes just down the required level. Once again, the peristaltic pump is run a little longer than the time required to collect the sample, and it will just bubble a little bit of the flushing air out of the reaction vessel the rest of the time (internal atmosphere is still flushed with reference air at a greater flow rate than the flow rate of the peristaltic pump in this mode).

The sample 73 is thus a perfectly homogenized (not just a theoretical approximated homogenate) of (i) the liquid tested medium water whose composition has been slightly modified during the previous reaction step and that was in contact with the chemical and/or biological system under study and (ii) the compensation solution that was just added. Once again, its amount is not estimated by flow rate measurement, but it is very accurately measured by weighting the sample later on. It is available for further chemical analysis (alkalinity, calcium, ammonium, nitrite, nitrate, orthophosphate, silicate, etc.).

If the compensation solution was correctly formulated, chemical composition of this sample should be identical or at least very close to targeted values in an ideally equilibrated chemostat. Since the composition of the compensation solution was anticipated using a dynamic mathematical model, any drift observed in the chemical composition of the sample denotes an over- or underestimation of that chemical by the model, we have here real-time validation of the model as a constituting part of the apparatus and/or method itself.

The sampling step (and the cycle) is finished when the unit is configured again as a closed unit in its reaction step configuration by stopping the peristaltic pump and inverting valves V2 and V3. A new cycle can then begin, which will use another of the four peristaltic pumps and compensation solution vessels, with another compensation solution ready to be injected.

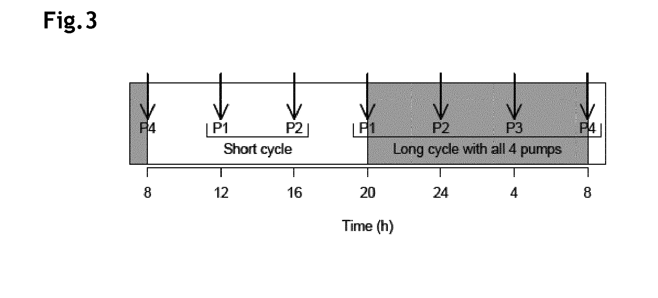

The whole system as illustrated in this embodiment thus has an autonomy of four cycles. In our prototype, we arrange the chemical and/or biological system so that each cycle could last for four hours without too great a change in the chemical composition of the liquid and gas test media inside the reaction vessel. That way, the unit has an autonomy of 16 hours outside of working hours, and it still satisfactorily approximates an ideal chemically static environment, despite corrections made at discrete time intervals. During working hours, two additional cycles are run with pumps P1 and P2 after collecting all samples and formulating other compensation solutions (see Fig 3). This concludes a 24 hour period of time. It leaves also enough working time for a technician to analyze all collected samples. Before the end of the working day, the two samples are collected and all compensation solutions are formulated for the next four cycles required to complete a 24 hour survey. This means we have six samples per 24 hours, and with a 12 hour photoperiod, we have two samples collected exactly when the light is switched on or off, two additional samples during the daylight and two additional samples during the dark phase. This configuration may be used to assess how the system behaves under a circadian cycle, including perhaps, nonlinear changes during day and/or night (a key feature of the device for biological systems with large variations that could be expected on a 24-hour cycle).

Fig 3 illustrates sampling intervals of 4 hours (configuration used in the prototype) which may provide sufficient time resolution on a circadian cycle to quantify how some processes, for example, photosynthesis, respiration, calcification, etc. vary during the day, including during light and dark phases. Using 4 separate pumps (each to complete one individual cycle), one can program a short cycle with 2 pumps P1 and P2 during working hours, and another, long cycle with all four pumps P1 - P4 until the next day. Arrows in Fig 3 show when samples are collected, and using which pump, at the end of each complete cycle. Compensation liquid solutions can also be calculated differently depending on the time of the day they will be added.

Note that the error in net fluxes measured over several cycles is no more than the addition of errors for each cycle, which are kept to the minimum by design. For a lower time resolution, but a higher precision over a longer period, several successive samples can be pooled and analyzed together. On the other hand, slight errors in the calculation by the model of the various compensation solutions do accumulate over successive cycles, leading to a better detection of even slight discrepancies between the model and the actual chemical and/or biological system under study. This design is also optimized to most accurately measure net fluxes and net balances that are usually measured by other means or by balancing equations containing several measurements, and where errors tend to accumulate to a much larger extent in the final estimation. For example, the crucial net flux of $CO_2$ between the liquid test medium reaction water and the air of the gas phase in the context of ocean acidification forecasts is not calculated here, but is quantitatively and most accurately measured directly in our artificial system by the IRGA $CO_2$ analyser once per cycle while operating its sampling step. Changes observed in air $P_{CO2}$ in the reaction vessel are the accumulations of air-water exchanges that occur all along one cycle (minus time required for addition - washing - sampling, that is, just 10 min in our prototype) when the unit is operated as a closed unit. Moreover, the same IRGA is used to selectively measure that air of the gas phase and reference gas alternatively, further minimising error in the quantification of $P_{CO2}$ changes between the two gasses.

The system is particularly adapted to the study of aquatic chemical and/or biological systems. Possible applications include, but are not limited to:

- Experimental setup to study chemical properties of calcium carbonate crystals in seawater. Chemistry of dissolution and precipitation of calcium carbonate in seawater is very complex and very far from an ideal system. There are several different forms of calcium carbonate crystals (aragonite, calcite, magnesian calcite; chemically, or biologically precipitated) and many ions that can interfere with the precipitation or dissolution of such crystals in seawater, including but not limited to, strontium or orthophosphates. Calcium carbonate is largely oversaturated (between 3 and 5 times) in surface seawater, but water acidification could lead to a dramatic change in the supersaturation state of seawater, or even to undersaturation in Antarctic waters near 2100. The system allows comfortable and accurate study the chemistry of calcium carbonate in seawater.

- The system may be used as a kind of sophisticated and ultra-precise respirometer. A respirometer is a chamber where respiration (and thus, metabolism) of living organisms is quantified. Simple respirometers are completely closed and allow measurement of respiration only over a short period of time (a complete survey over 24h is not possible). More complex respirometers operate in open-systems, but are much less accurate because they suffer from the same sources of measurement errors as traditional chemostats (e.g. estimation of oxygen consumed depends on the precision in the estimation of flow rates, and on the false assumption that the solution is instantaneously a perfect mix in the respirometer). In comparison, the invention may be used for both accurate measurement of oxygen consumption (or carbon dioxide production) by respiration, and repeated measurements along a 24h period of time, or even longer.

- The system may be used as an experimental device for research on the impact of ocean acidification on living organisms, the application it was primarily designed for. All parameters like volume and size of the unit, volume of the entrapped atmosphere gas phase, time between two successive cycles and volume of each addition and sample are subject to appropriate selection according to the biological system under study.

- The system can be run to culture bacteria, protists, fungi, phytoplankton, zooplankton, ..., in well-specified physico-chemical conditions and to monitor their growth and phenotypical changes in these conditions. Two main targeted applications can be envisioned: (1) as in previous applications the device can be used as an experimental unit for research in ecophysiological studies, and (2) the device can be used to accurately measure the way the biological system operates in order to better design industrial chemostats, thanks to complete and accurate characterization of fluxes between the biological system, liquid and gas media.

- The system may be also used to culture cells in suspended liquid medium wherever accurate quantification of exchanges between these cells and the liquid medium and/or the gas phase are required. This is useful in experimental situations in the fields of physiology, medicine, veterinary sciences, or ecology.

- The biological system can also comprise sediments, and in this case, exchanges between the sediments, the living organisms and the liquid medium can be studied. For instance, fluxes between sediments coming from the seabed, or from the bottom of a river or a lake and the water can be characterized. This can also include ecotoxicological studies where sequestration or liberation of toxic substances, like heavy metals, PCBs, hydrocarbons, etc. inside the sediments and/or the living organisms can be precisely assessed using such a device.

- Stable or radioisotopes can also be used in the compensation solutions and/or in the reference gas in order to better track specific fluxes in the system. For example, the reference gas may be composed of air where $^{14}N_2$ (at more than 99% in air) is replaced by $^{15}N_2$. Fluxes of $^{15}N_2$ inside the biological organisms quantify nitrogen fixation. On the other hand, Fluxes of $^{14}N_2$ in the air quantify denitrification processes. Both fluxes are measurable in the present device, allowing for a direct measurement of nitrogen fixation or denitrification, a task hardly possible currently with another system.

Further examples of industrial applicability of the system will be immediately apparent to the skilled addressee.

For example, one of the consequences of the increase of $CO_2$ in the atmosphere is a shift of inorganic carbon equilibrium in the oceans, leading to so-called ocean acidification (partial neutralisation of alkaline seawater pH). Since the beginning of industrial era, mean seawater pH has already lowered by about 0.1 units, and a further decrease by 0.2-0.3 units is expected by the next century, or so. Whether this pH shift has an impact on aquatic live being is sure now. It impacts calcification rate, photosynthesis rate, as well as, other biochemical reactions that are sensitive to extracellular pH. The calcium carbonate (both aragonite and calcite) saturation horizon is the depth where corresponding form of calcium carbonate is in equilibrium in seawater. Above, water is supersaturated and favours precipitation (mainly by accretion by living organisms). Below, calcium carbonates dissolves by chemical erosion in the depths of the oceans. These saturation horizons occur at lower and lower depths as pH of seawater drops. Due to the large number of potential effects on the physiology of marine plants, animals and microbes, their indirect effects on whole ecosystems, and changes in global chemical state regarding precipitation and dissolution of calcium carbonate, it is not clear yet how aquatic environments will be impacted at large scale by this pH drop. This is currently the field of intensive research by the scientific community.

In this context, it is important to know which system acts as a sink (it consumes more inorganic carbon than it produces) or as a source (the opposite). Indeed, possible actions to limit $CO_2$ increase in the atmosphere include any resources management that favor sinks against sources of inorganic carbon on the earth. This requires quantification and fine understanding of the carbon cycle on our planet. Whether a biological and/or chemical system acts as a sink or a source of inorganic carbon is not easy to quantify, because this is the result of several fluxes of carbon in opposite directions (biological systems, in particular, are very dynamics). Net balance is often quantified with a large part of uncertainty, as the net result of many fluxes that are individually quantified with significant error (photosynthesis, respiration, calcification, nitrification, denitrification, chemical precipitation or dissolution, ...). These errors accumulate in the equations.

The most accurate quantification of the net carbon fluxes of a given system is by direct measurement. For aquatic systems, the system of the present invention may be used to place the chemical or biological aquatic system under study in such conditions that net resulting carbon exchanges at the air-water interface can be directly and accurately measured.

Another aim of the system is to permit operation as a chemostat (chemical environment is static), with a control of inorganic composition of the water, its pH, and its nutrients load (for biological systems).

A third aim is to allow concurrent determination of most important mechanisms that alter inorganic carbon chemistry of the water and its pH in natural systems, i.e., precipitation or dissolution of calcium carbonate, photosynthesis and respiration, nitrification and denitrification, exchange at air-water interface, ... The system of the present invention may be used to target measurement of these mechanisms simultaneously, with the highest possible precision, non-destructively, and without using radioactive isotopes (for example $^{14}$C incorporation measurements). Temporal resolution is crucial too: the system must remain stable enough for a few days and several measurements per day should allow to understand circadian changes in the processes.

A fourth aim is to get a good mechanistic understanding of the processes that occur in the unit. The approach that is made possible in accordance with the invention may be used to reverse the usual mode of operation between an experimental system and its mathematical (dynamic) model. Usually, an experimental system is studied first. Being a chemostat, it requires stabilisation of the chemical composition of the medium. This is done by measurements in the outlet water, and adjustment of the inlet water to stabilize concentration of the chemicals inside the unit, independently from the mathematical model. The model is eventually adjusted on the experimental data, but it never interacts directly with the way the experimental unit (the chemostat) is operating. The model is thus a kind of passive mathematical representation of the changes that occur independently in the experimental unit. On the contrary to an usual experimental unit, the system of the present invention may be (and in some configurations must be) directly driven by the mathematical model. The latter is used to anticipate changes that will occur in the system a few hours ahead, and to formulate supplementations in order to bring required corrections. Compatibility of the model with the real system under study is quantified by the variations observed with time that are due to incorrect prediction by the model. In such a case, the model must be corrected first, and the experiment in the system is run again until the whole system stabilizes. At that moment, we obtain a direct experimental validation of the mathematical model, since this demonstrates that changes in the system are correctly predicted and anticipated in the calculations.

**Claims**

1.  A method of analysing behaviour of a system comprising a chemical and/or biological sample in a liquid medium, the method comprising:

    a first cycle comprising:

        - a first reaction step comprising allowing the chemical and/or biological sample to react in a reaction vessel for a first reaction step time (t1) in a closed system in a liquid medium; and
        - a first sampling step comprising the sequential steps of: (i) combining and substantially homogenising a first compensation liquid of known, predetermined weight and composition with the liquid medium; and (ii) removing a portion of the homogenised liquid medium for analysis; and

    a second cycle comprising

        - a second reaction step comprising allowing the chemical and/or biological sample to react in the reaction vessel for a second reaction step time (t2) in a closed system in the liquid medium resulting from the first cycle; and
- a second sampling step comprising the sequential steps of: (i) combining and substantially homogenising a second compensation liquid of known, predetermined weight and composition with the liquid medium from the second cycle; and (ii) removing a portion of the homogenised liquid medium for analysis.

2. A method of analysing behaviour of a system in accordance with claim 1, in which the composition of at least one of the compensation liquids is selected so as to match an anticipated composition of the liquid medium at the time at which the compensation liquid and the liquid medium are combined, the said composition of the compensation liquid being determined by modelling.

3. A method of analysing behaviour of a system in accordance with claim 2, in which the modelling of the composition of the compensation liquid includes use of data derived from analysis of the liquid medium from a previous sampling step.

4. A method of analysing behaviour of a system in accordance with any preceding claim, in which the liquid medium contacts a reference gas during the reaction step.

5. A method of analysing behaviour of a system in accordance with claim 4, in which (i) during the reaction step the reference gas is provided in a closed system and in which (ii) the method comprises analysing at least one property of the reference gas in a way adapted to determine possible changes in that property induced during the reaction step.

6. A method of analysing behaviour of a system in accordance with any preceding claim, in which the behaviour of the system is analysed by weighing each liquid which is added to and removed from the reaction vessel.

7. An apparatus for analysing behaviour of a system comprising a chemical and/or biological sample in a liquid medium, the apparatus being adapted to be switched between operation in a closed loop reaction cycle and operation in a sampling cycle, the apparatus comprising:

   a reaction vessel adapted to be maintained in a controlled environment and adapted to contain the chemical and/or biological sample in the liquid medium;
   a gas circulation system adapted to admit a reference gas to the system prior to a reaction cycle, to circulate the gas in a closed loop in the system during the reaction cycle so that the gas contacts the liquid medium and to supply a portion of the circulated gas for analysis during the sampling cycle;
   at least one compensation liquid vessel adapted to contain a compensation liquid to be combined with the liquid medium during the sampling cycle, the compensation liquid vessel being adapted to be supplied with the reference gas;
   a liquid combining system adapted to combine and substantially homogenize the compensation liquid and the liquid medium as part of the sampling cycle;
   a liquid sampling system adapted to remove a sample portion from the liquid medium as part of the sampling cycle;
   at least one liquid sample receiving vessel adapted to receive the sample portion of the liquid medium as part of the sampling cycle; and
   a controller adapted to switch the system between its reaction cycle and its sampling cycle.

8. An apparatus in accordance with claim 7, in which the same vessel is adapted to serve as a combined compensation liquid vessel and liquid sample receiving vessel.

9. An apparatus in accordance with claim 7 or claim 8, in which the apparatus comprises a plurality of a compensation liquid vessel and liquid sample receiving vessel or a plurality of combined compensation liquid vessel and liquid sample receiving vessel, and in which the apparatus is configured such that each liquid sample receiving vessel is adapted to receive a liquid sample from a different reaction cycle of the apparatus.

10. An apparatus in accordance with any of claims 7 to 9, in which a liquid sampling system comprises one or more pumps having associated supply lines, each pump and its associated supply lines being configured to transfer liquid between the reaction vessel and a single liquid sample receiving vessel.

11. An apparatus in accordance with any of claims 7 to 10, in which the apparatus further comprises at least one probe, notably a pH probe and/or an oxygen probe, adapted to measure a characteristic of the liquid medium.

12. A conglomerated apparatus comprising a plurality of apparatus in accordance with any of claims 7 to 11, in which the conglomerated apparatus comprises a reaction vessel holder adapted to retain the reaction vessels of each apparatus in a configuration such that each reaction vessel is subjected to substantially identical external conditions.

13. An apparatus in accordance with claim 12, in which the gas circulation system is adapted to selectively supply a portion of the circulated gas from each individual apparatus to a single gas analyser during the sampling cycle of that individual apparatus.

14. Use of a method in accordance with any or claims 1 to 6 and/or an apparatus in accordance with any of claims 7 to 13 to analyse a system selected from the group consisting of a system configured to analyse the impact of ocean acidification on a living organism, a system configured to quantify oxygen consumption and/or carbon dioxide production of a living organism, and a system for studying chemical properties of calcium carbonate crystals in seawater.

**Patentansprüche**

1. Verfahren zur Analyse des Verhaltens eines Systems, umfassend eine chemische und/oder biologische Probe in einem flüssigen Medium, wobei das Verfahren umfasst:

   einen ersten Kreislauf, umfassend:

   - einen ersten Reaktionsschritt, umfassend Reagierenlassen der chemischen und/oder biologischen Probe in einem Reaktionsgefäß für eine erste Reaktionsschrittzeit (t1) in einem geschlossenen System in einem flüssigen Medium; und
   - einen ersten Probeentnahmeschritt, umfassend die aufeinanderfolgenden Schritte: (i) Vereinigen und im Wesentlichen Homogenisieren einer ersten Ausgleichsflüssigkeit bekannten, vorher bestimmten Gewichts und Zusammensetzung mit dem flüssigen Medium; und (ii) Entfernen eines Teils des homogenisierten, flüssigen Mediums zur Analyse; und

   einen zweiten Kreislauf, umfassend:

   - einen zweiten Reaktionsschritt, umfassend Reagierenlassen der chemischen und/oder biologischen Probe in dem Reaktionsgefäß für eine zweite Reaktionsschrittzeit (t2) in einem geschlossenen System in dem flüssigen Medium, resultierend aus dem ersten Kreislauf; und
   - einen zweiten Probeentnahmeschritt, umfassend die aufeinanderfolgenden Schritte: (i) Vereinigen und im Wesentlichen Homogenisieren einer zweiten Ausgleichsflüssigkeit bekannten, vorher bestimmten Gewichts und Zusammensetzung mit dem flüssigen Medium aus dem zweiten Kreislauf; und (ii) Entfernen eines Teils des homogenisierten, flüssigen Mediums zur Analyse.

2. Verfahren zur Analyse des Verhaltens eines Systems nach Anspruch 1, in dem die Zusammensetzung von mindestens einer der Ausgleichsflüssigkeiten so ausgewählt wird, dass sie einer berechneten Zusammensetzung des flüssigen Mediums zum Zeitpunkt, an dem die Ausgleichsflüssigkeit und das flüssige Medium vereinigt werden, entspricht, wobei die Zusammensetzung der Ausgleichsflüssigkeit durch Modellieren bestimmt wird.

3. Verfahren zur Analyse des Verhaltens eines Systems nach Anspruch 2, in dem das Modellieren der Zusammensetzung der Ausgleichsflüssigkeit die Verwendung von Daten, abgeleitet aus der Analyse des flüssigen Mediums in einem vorherigen Probeentnahmeschritt, beinhaltet.

4. Verfahren zur Analyse des Verhaltens eines Systems nach einem der vorausgehenden Ansprüche, in dem das flüssige Medium ein Vergleichsgas während des Reaktionsschritts berührt.

5. Verfahren zur Analyse des Verhaltens eines Systems nach Anspruch 4, in dem (i) während des Reaktionsschritts das Vergleichsgas in einem geschlossenen System bereitgestellt wird, und in dem (ii) das Verfahren das Analysieren von mindestens einer Eigenschaft des Vergleichsgases derart eingerichtet umfasst, um mögliche Veränderungen dieser Eigenschaft, hervorgerufen während des Reaktionsschritts, zu bestimmen.

6. Verfahren zur Analyse des Verhaltens eines Systems nach einem der vorausgehenden Ansprüche, in dem das Verhalten des Systems durch Wiegen jeder Flüssigkeit, die hinzugefügt oder aus dem Reaktionsgefäß entfernt wird, analysiert wird.

7. Vorrichtung zur Analyse des Verhaltens eines Systems, umfassend eine chemische und/oder biologische Probe in einem flüssigen Medium, wobei die Vorrichtung eingerichtet ist, zwischen Betrieb in einem geschlossenen Schleifenreaktionskreislauf und Betrieb in einem Probeentnahmekreislauf umgeschaltet zu werden, wobei die Vorrichtung umfasst:

ein Reaktionsgefäß, eingerichtet, um in einer kontrollierten Umgebung gehalten zu werden, und eingerichtet, um die chemische und/oder biologische Probe in dem flüssigen Medium zu fassen;

ein Gaskreislaufsystem, eingerichtet, um ein Vergleichsgas in das System vor einem Reaktionskreislauf einzulassen, das Gas in einer geschlossenen Schleife in dem System während des Reaktionskreislaufs zu zirkulieren, so dass das Gas das flüssige Medium berührt, und einen Teil des zirkulierten Gases zur Analyse während des Probeentnahmekreislaufs bereitzustellen;

mindestens ein Ausgleichsflüssigkeitsgefäß, eingerichtet, um eine Ausgleichsflüssigkeit zu fassen, die mit dem flüssigen Medium während des Probeentnahmekreislaufs vereinigt wird, wobei das Ausgleichsflüssigkeitsgefäß eingerichtet ist, um mit dem Vergleichsgas gespeist zu werden;

ein Flüssigkeitsvereinigungssystem, eingerichtet, um die Ausgleichsflüssigkeit und das flüssige Medium als Teil des Probeentnahmekreislaufs zu vereinigen und im Wesentlichen zu homogenisieren;

ein Flüssigkeitsprobeentnahmesystem, eingerichtet, um einen Probeteil aus dem flüssigen Medium als Teil des Probeentnahmekreislaufs zu entfernen;

mindestens ein Flüssigkeitsprobeaufnahmegefäß, eingerichtet, um den Probenteil des flüssigen Mediums als Teil des Probeentnahmekreislaufs aufzunehmen; und

einen Schalter, eingerichtet, um das System zwischen seinem Reaktionskreislauf und seinem Probeentnahmekreislauf umzuschalten.

8. Vorrichtung nach Anspruch 7, in der dasselbe Gefäß eingerichtet ist, um als ein kombiniertes Ausgleichsflüssigkeitsgefäß und Flüssigkeitsprobeaufnahmegefäß zu dienen.

9. Vorrichtung nach Anspruch 7 oder Anspruch 8, in der die Vorrichtung eine Vielzahl eines Ausgleichsflüssigkeitsgefäßes und Flüssigkeitsprobeaufnahmegefäßes oder eine Vielzahl des kombinierten Ausgleichsflüssigkeitsgefäßes und Flüssigkeitsprobeaufnahmegefäßes umfasst, und in der die Vorrichtung so konfiguriert ist, dass jedes Flüssigkeitsprobeaufnahmegefäß eingerichtet ist, um eine Flüssigkeitsprobe aus einem unterschiedlichen Reaktionskreislauf der Vorrichtung aufzunehmen.

10. Vorrichtung nach einem der Ansprüche 7 bis 9, in der ein Flüssigkeitsprobeentnahmesystem eine oder mehrere Pumpen mit angeschlossenen Versorgungsleitungen umfasst, wobei jede Pumpe und ihre angeschlossenen Versorgungsleitungen konfiguriert sind, um Flüssigkeit zwischen dem Reaktionsgefäß und einem einzelnen Flüssigkeitsprobeaufnahmegefäß zu übertragen.

11. Vorrichtung nach einem der Ansprüche 7 bis 10, in der die Vorrichtung weiter mindestens eine Sonde, insbesondere eine pH-Sonde und/oder eine Sauerstoff-Sonde umfasst, die eingerichtet ist, eine Eigenschaft des flüssigen Mediums zu messen.

12. Zusammengesetzte Vorrichtung, umfassend eine Vielzahl der Vorrichtung nach einem der Ansprüche 7 bis 11, in der die zusammengesetzte Vorrichtung einen Reaktionsgefäßhalter umfasst, der eingerichtet ist, um die Reaktionsgefäße jeder Vorrichtung in einer Konfiguration zu halten, so dass jedes Reaktionsgefäß im Wesentlichen identischen äußeren Bedingungen unterworfen ist.

13. Vorrichtung nach Anspruch 12, in der das Gaskreislaufsystem eingerichtet ist, um selektiv einen Teil des zirkulierten Gases aus jeder einzelnen Vorrichtung an einen einzelnen Gasanalysierer während des Probeentnahmekreislaufs der einzelnen Vorrichtung bereitzustellen.

14. Verwendung eines Verfahrens nach einem der Ansprüche 1 bis 6 und/oder einer Vorrichtung nach einem der Ansprüche 7 bis 13, um ein System, ausgewählt aus der Gruppe, bestehend aus einem System, konfiguriert, um die Auswirkung der Versauerung der Meere auf ein Lebewesen zu analysieren, einem System, konfiguriert, um den Sauerstoffverbrauch und/oder die Kohlenstoffdioxidproduktion eines Lebewesens zu messen, und einem System zur Untersuchung der chemischen Eigenschaften von Calciumcarbonatkristallen in Meerwasser, zu analysieren.

**Revendications**

1. Procédé d'analyse du comportement d'un système comprenant un échantillon chimique et/ou biologique dans un milieu liquide, le procédé comprenant :

   un premier cycle comprenant :

   - une première étape réactionnelle comprenant le fait de permettre à l'échantillon chimique et/ou biologique de réagir dans un récipient réactionnel pendant une durée de première étape réactionnelle (t1) dans un système fermé dans un milieu liquide, et
   - une première étape d'échantillonnage comprenant les étapes séquentielles de : (1) combinaison et homogénéisation substantielle d'un premier liquide de compensation de poids et composition connus et prédéterminés avec le milieu liquide et (ii) prélèvement d'une partie du milieu liquide homogénéisé pour analyse, et

   un deuxième cycle comprenant :

   - une deuxième étape réactionnelle comprenant le fait de permettre à l'échantillon chimique et/ou biologique de réagir dans le récipient réactionnel pendant une durée de deuxième étape réactionnelle (t2) dans un système fermé dans le milieu liquide résultant du premier cycle, et
   - une deuxième étape d'échantillonnage comprenant les étapes séquentielles de : (1) combinaison et homogénéisation substantielle d'un deuxième liquide de compensation de poids et composition connus et prédéterminés, avec le milieu liquide du deuxième cycle, et (ii) prélèvement d'une partie du milieu liquide homogénéisé pour analyse.

2. Procédé d'analyse du comportement d'un système selon la revendication 1, dans lequel la composition d'au moins un des liquides de compensation est choisie de sorte qu'elle corresponde à la composition anticipée du milieu liquide au moment auquel le liquide de compensation et le milieu liquide sont combiné, ladite composition du liquide de compensation étant déterminée par modélisation.

3. Procédé d'analyse du comportement d'un système selon la revendication 2, dans lequel la modélisation de la composition du liquide de compensation comprend l'utilisation de données dérivées de l'analyse du milieu liquide dans une étape d'échantillonnage précédente.

4. Procédé d'analyse du comportement d'un système selon l'une quelconque des revendications précédentes, dans lequel le milieu liquide est en contact avec un gaz de référence pendant l'étape réactionnelle.

5. Procédé d'analyse du comportement d'un système selon la revendication 4, dans lequel (i) pendant l'étape réactionnelle, le gaz de référence est dans un système fermé, et dans lequel (ii) le procédé comprend l'analyse d'au moins une propriété du gaz de référence d'une manière adaptée pour déterminer les changements possibles de cette propriété induits pendant l'étape réactionnelle.

6. Procédé d'analyse du comportement d'un système selon l'une quelconque des revendications précédentes, dans lequel le comportement du système est analysé en pesant chaque liquide qui est ajouté et prélevé du récipient de réaction.

7. Appareil d'analyse du comportement d'un système comprenant un échantillon chimique et/ou biologique, l'appareil étant adapté pour pouvoir passer d'une opération d'un cycle réactionnel en boucle fermé à une opération d'un cycle d'échantillonnage, l'appareil comprenant :

   un récipient de réaction adapté à être maintenu dans un environnement contrôlé et adapté à contenir l'échantillon chimique et/ou biologique dans le milieu liquide ;
   un système de circulation de gaz adapté à faire entrer un gaz de référence dans le système avant un cycle réactionnel, faire circuler le gaz dans une boucle fermée dans le système pendant le cycle réactionnel de sorte que le gaz entre en contact avec le milieu liquide, et amener une partie du gaz en circulation pour analyse pendant le cycle d'échantillonnage ;
   au moins un récipient de liquide de compensation adapté à contenir un liquide de compensation à combiner au milieu liquide pendant le cycle d'échantillonnage, le récipient du liquide de compensation étant adapté à être alimenté en gaz de référence ;

un système de combinaison de liquides adapté à combiner et substantiellement homogénéiser le liquide de compensation et le milieu liquide comme partie du cycle d'échantillonnage ;

un système d'échantillonnage de liquide adapté à prélever une partie d'échantillon du milieu liquide comme partie du cycle d'échantillonnage ;

au moins un récipient de réception de l'échantillon liquide adapté à réceptionner la partie d'échantillon du milieu liquide comme partie du cycle d'échantillonnage, et

un contrôleur adapté à commuter le système entre son cycle réactionnel et son cycle d'échantillonnage.

8. Appareil selon la revendication 7, dans lequel le même récipient est adapté à servir de récipient du liquide de compensation et de récipient de réception de l'échantillon liquide combiné.

9. Appareil selon la revendication 7 ou 8, dans lequel l'appareil comprend une série de récipient du liquide de compensation et de récipient de réception de l'échantillon liquide ou une série de récipient du liquide de compensation et récipient de réception de l'échantillon liquide combiné, et dans lequel l'appareil est configuré pour que chaque récipient de réception de l'échantillon liquide soit adapté à recevoir un échantillon liquide d'un cycle réactionnel différent de l'appareil.

10. Appareil selon l'une quelconque des revendications 7 à 9, dans lequel un système d'échantillonnage de liquide comprend une ou plusieurs pompes ayant les tubages associés, chaque pompe et son tubage associé étant configurée pour transférer un liquide entre le récipient réactionnel et un seul récipient de réception de l'échantillon liquide.

11. Appareil selon l'une quelconque des revendications 7 à 10, dans lequel l'appareil comprend en outre, au moins une sonde, notamment une sonde de pH et/ou une sonde à oxygène, adapté à mesurer une caractéristique du milieu liquide.

12. Appareil congloméré comprenant une série d'appareils selon l'une quelconque des revendications 7 à 11, dans lequel l'appareil congloméré comprend un support de récipient réactionnel adapté à retenir les récipients réactionnels de chaque appareil en une configuration telle que chaque récipient réactionnel est soumis à des conditions externes substantiellement identiques.

13. Appareil selon la revendication 12, dans lequel le système de circulation de gaz est adapté à alimenter sélectivement une partie du gaz en circulation de chaque appareil individuel vers un seul analyseur de gaz pendant le cycle d'échantillonnage de cet appareil individuel.

14. Utilisation d'un procédé selon l'une quelconque des revendications 1 à 6 et/ou d'un appareil selon l'une quelconque des revendications 7 à 13, pour analyser un système choisi parmi le groupe consistant en un système configuré pour analyser l'impact de l'acidification des océans sur un organisme vivant, un système configuré pour quantifier la consommation d'oxygène et/ou la production de dioxyde de carbone d'un organisme vivant, et un système pour étudier les propriétés chimiques de cristaux de carbonate de calcium dans l'eau de mer.

**Fig. 1**

# Fig.2

**Fig 2a**

**Fig 2b**

**Fig 2d**

**Fig 2c**

# Fig.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010110773 A1
- US 20040024493 A1
- CN 101709263 A